# EUROPEAN PATENT APPLICATION

(11) **EP 0 530 901 A1**
(43) Date of publication of application: **10.03.1993**
(21) Application number: 92202603.4
(22) Date of filing: 27.08.1992
(51) Int. Cl.: C07D 493/22, A01N 43/90, A61K 31/365

(54) **13-beta-O-methoxymethyl-22,23-dihydro avermectin B1A/B1B aglycone**

(30) Priority: 04.09.1991 US 754838
(71) Applicant: MERCK & CO. INC., Rahway New Jersey 07065-0900 (US)
(72) Inventor: Linn, Bruce O., Bridgewater, NJ 08807 (US); Mrozik, Helmut, Matawan, NJ 07747 (US)
(74) Representative: Thompson, John Dr.

(57) **Abstract**

The novel compound 13β-O-methoxymethyl-22,23-dihydro avermectin B1a/B1b aglycone is useful as an anthelmintic and antiparasitic agent and has been discovered to be unexpectedly superior to the corresponding 13α-compound. The compound is also useful as a pesticide and insecticide against agricultural pests. Compositions containing said compound and methods of administering said compositions are also disclosed.

## Description

### BACKGROUND OF THE INVENTION

The invention relates to a novel substituted 13β-methoxymethyl avermectin aglycone derivative useful as a antiparasitic agent and to the novel intermediates useful in the process for preparing said derivative. This invention also relates to compositions of said derivatives and methods of administering said compositions.

The term avermectin (previously referred to as C-076) is used to describe a series of compounds isolated from the fermentation broth of an avermectin producing strain of Streptomyces avermitilis and derivatives thereof. The morphological characteristics of the culture are completely described in U.S. Patent No. 4,310,519 and are incorporated herein by reference. The avermectin compounds are a series of macrolides, each of which is substituted thereon at the 13-position with a 4'-(α-L-oleandrosyl)-α-L-oleandrose group. Avermectin compounds and the derivatives thereof of this invention have a very high degree of anthelmintic and antiparasitic activity.

### DESCRIPTION OF THE PRIOR ART

The avermectin series of compounds from which the derivatives of the invention are derived have the following structure: 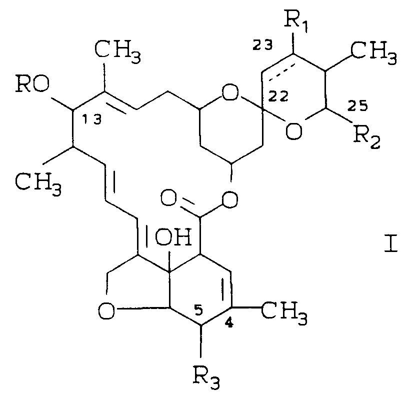
wherein R is the 4'-(α-1-oleandrosyl)-α-1-oleandrose group of the structure: 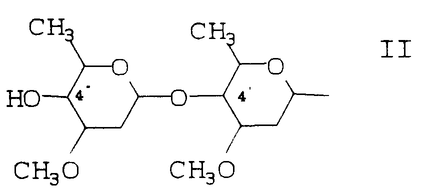
and wherein the broken line indicates a single or a double bond; R₁ is hydroxy and is present only when said broken line indicates a single bond;
- R₂: is iso-propyl or sec-butyl; and
- R₃: is methoxy or hydroxy.

There are eight different major avermectin natural product compounds and they are given the designations A1a, A1b, A2a, A2b, B1a, B1b, B2a and B2b based upon the structure of the individual compounds.

In the foregoing structural formula, the individual avermectin compounds are as set forth below. (The R group is 4'-(α-L-oleandrosyl)-α-L-oleandrose):

| | R₁ | R₂ | R₃ |
|---|---|---|---|
| A1a | (22,23-Double Bond) | sec-butyl | -OCH₃ |
| A1b | (22,23-Double Bond) | iso-propyl | -OCH₃ |
| A2a | -OH | sec-butyl | -OCH₃ |
| A2b | -OH | iso-propyl | -OCH₃ |
| B1a | (22,23-Double Bond) | sec-butyl | -OH |
| B1b | (22,23-Double Bond) | iso-propyl | -OH |
| B2a | -OH | sec-butyl | -OH |
| B2b | -OH | iso-propyl | -OH |

The avermectin compounds are generally isolated as mixtures of homologous a and b components. Such compounds differ only in the nature of the R₂ substituent and the minor structural difference has been found to have very little effect on the isolation procedures, chemical reactivity and biological activity of such compounds.

In the isolation of the avermectin compounds from the fermentation broth, which serve as starting materials for the instant processes, the various avermectin compounds will be found to have been prepared in unequal amounts. In particular an "a" series compound will be prepared in a higher proportion than the corresponding "b" series compound. The difference between the "a" series and "b" series is constant throughout the avermectin compounds and consists of a sec-butyl group and an iso-propyl group respectively at the 25-position. This difference, of course, does not interfere with any of the instant reactions. In particular it may not be necessary to separate the "b" component from the related "a" component. Separation of these closely related compounds is often not practiced since the "b" compound often is present only in a small amount, and the structural difference has negligible effect on the reaction processes and biological activities.

In particular it has been found that the starting materials for the compounds of this invention are conveniently prepared in a ratio of about 80% to 95% avermectin B1a or A1a and less than 20% avermectin B1b or A1b. Thus the preferred composition of this invention is one which contains not less than 80% of the "a" component and not more than 20% of the "b" component.

US Patent 4,587,274 to Linn and Mrozik discloses 13-polyalkoxy avermectin derivatives and in particular discloses the 13-methoxymethyl avermectin B1a/B1b aglycone compound and processes for the preparation thereof. The sterochemistry of and compound is not specified and then is no specific disclosures for the preparation of 13β-O-methoxymethyl-22,23-dihydro avermectin B1a/B1b. The unexpectedly superior properties of the instant compound are not suggested.

### SUMMARY OF THE INVENTION

The invention relates to a novel substituted 13-(alkoxy)methyl derivatives of the avermectin aglycones, specifically the 13-β-O-methoxy methyl derivative, and processes for preparing the same. The sugar portion of avermectins is replaced with a methoxymethyl group in the β-position

Accordingly, it is an object of the invention to provide a novel substituted and unsubstituted 13β-O-methoxymethyl derivative of the avermectin B1a/B1b aglycone that is useful as an antiparasitic agent.

A further object of the invention is to provide processes for preparation of said novel compound.

Another object of the invention is to provide pharmaceutical compositions for administering said novel compound.

Still another object of the invention is to provide for such compound which is useful as an insecticide and pesticide against agricultural pests.

A still further object of the invention is to provide methods for the treatment of animals suffering from parasitic conditions.

These and other objects and advantages of the present invention will become apparent from the following description.

### DESCRIPTION OF THE INVENTION

The compound of this invention has the following structural formula: 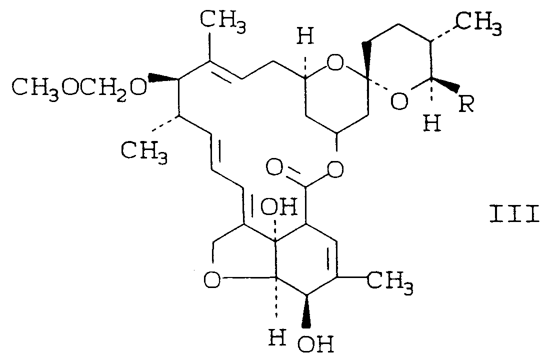
where R is sec-butyl or iso-propyl.

The above pair of homologous compounds has been found to have surprising activity as an anthelmintic and antiparasitic agent. In particular, the compounds have an unexpectedly long duration of activity against ectoparasites of domestic animals. When the instant compound or combination of the "a" and "b" compound is tested on dogs as a topical antiparasitic agent against the common dog flea, a single treatment is seen to provide complete protection against repeated challenges of flea infestation for a period of up to 6 weeks duration. In comparison, the corresponding 13-α-compound offers protection for less than 4 weeks.

### PREPARATION OF STARTING MATERIALS

The ultimate starting materials for the compounds of this invention are the avermectin fermentation products defined above specifically avermectin B1a/B1b. Thus it is apparent that additional reactions are required to prepare the instant compounds. Specifically, reactions are carried out at the 13, and 22,23-positions. It is generally preferred to prepare whatever substituents are required at these positions before carrying out the reaction to introduce the β-methoxymethyl 13-position group on the substrate. Such a procedure generally avoids undesirable side reactions. This technique is not required, however, and if desired, other sequences may be used. In addition, it is often necessary to protect certain reactive hydroxy groups where reaction with the above reagents is not desired. With the appropriate positions protected, the above reactions may be carried out without affecting the remainder of the molecule. Subsequent to any of the above described reactions the protecting group may be removed and the unprotected product isolated. The protecting group employed is ideally one which may be readily synthesized, will not be affected by the reaction with halomethyl ether reagents and may be readily removed without affecting any other functions of the molecule. It is noted that the instant protected compounds are novel and have considerable antiparasitic activity. They are included within the ambit of the instant invention. One preferred type of protecting group for the avermectin type of molecule is the tri-substituted silyl group, preferably the trialkyl silyl group. One especially preferred example is the t-butyl dimethylsilyl group. The reaction preparing the protected compound is carried out by reacting the hydroxy compound with the appropriately substituted silylhalide, preferably the silylchloride in an aprotic polar solvent such as dimethylformamide. Imidazole is added as a catalyst. The reaction is complete in from 1 to 24 hours at from 0 to 25°C. For the 5-position hydroxy group the reaction is complete in from 1/2 to 3 hours at from 0°C to room temperature. This reaction is selective to the 5 position under the conditions above described and very little, if any, silylation is observed at other hydroxy substituted positions.

The silyl group may be removed after the other contemplated reactions may be carried out. The silyl group or groups are removed by stirring the silyl compound in methanol catalyzed by a catalytic amount of an acid, preferably a sulfonic acid such as p-toluenesulfonic acid. The reaction is complete in about 1 to 12 hours at from 0° to 50°C.

Another of the starting materials used in the foregoing reaction scheme are those in which the 22,23 double bond of the B1 compounds has been reduced to a single bond. As is readily apparent from an analysis of the structure of avermectin starting materials there are 5 unsaturations in the 1-series of compounds. Thus in the "1" series of compounds it is necessary to reduce the 22,23 double bond while not affecting the remaining four unsaturations or any other functional group present on the molecule in order to selectively prepare the 22,23 dihydro avermectins. It is necessary to select a specific catalyst for the hydrogenation, one that will selectively hydrogenate the least hindered from among a series of unsaturations. The preferred catalyst for such a selective hydrogenation procedure is one having the formula:

[(Ph₃P)₃RhZ)]

wherein
Ph is phenyl and Z is halogen. The reduction procedure is completely described in U.S. Patent 4,199,569 to Chabala et al.

All of the avermectin starting materials for the compounds of this invention require the removal of both of the α-L-oleandrosyl moieties (described in U.S. Patent 4,206,205 to Mrozik et al.).

The reaction conditions which are generally applicable to the preparation of the aglycone involve dissolving the avermectin compound or the hydrogenated avermectin compound in an aqueous acidic non-nucleophilic organic solvent, miscible with water, preferably dioxane, tetrahydrofuran, dimethoxyethane, dimethylformamide, bis-2-methoxyethyl ether, and the like, in which the water concentration is from 0.1 to 20% by volume. Concentrated acid is added to the aqueous organic solvent to the extent of 1 to 10% by volume. The reaction mixture is generally stirred at about 20-40°C, preferably at room temperature, for from 6 to 24 hours. The products are isolated, and mixtures are separated by techniques such as column, thin layer, preparative and high pressure liquid chromatography, and other known techniques.

The acids which may be employed in the above process include mineral acids and organic acids such as sulfuric, hydrohalic, phosphoric, trifluoroacetic, trifluoro methane sulfonic and the like. The hydrohalic acids are preferably hydrochloric or hydrobromic. The preferred acid in the above process is sulfuric acid.

A further procedure for the preparation of the aglycone of the avermectin compounds or of the hydrogenated avermectin compounds utilizes a different solvent system. For the preparation of the aglycone, 1% acid, by volume, in methanol under the foregoing reaction conditions has been found to be appropriate.

When this procedure is employed on the starting materials containing the 22,23-double bond, there is a possibility of an acid catalyzed addition of the solvent to the double bond. If such occurs, chromatographic purification will remove the by-product in order to allow for further reactions.

The acids listed above are appropriate for this process, and again sulfuric acid is the preferred acid.

### DISCUSSION OF CHEMICAL REACTIONS

The instant compound is prepared by reacting the appropriately protected avermectin aglycones, wherein the 13-position substituent is hydroxy, with a halomethyl ether, specifically chloromethyl methyl ether in the presence of a non-reactive acid acceptor such as a sterically hindered tertiary amine in a dry aprotic solvent. The process is outlined in the following general reaction scheme I which for clarity, shows the partial structural formula including only the 10, 11, 12, 13, 14 and 15 ring carbon atoms of the formula: 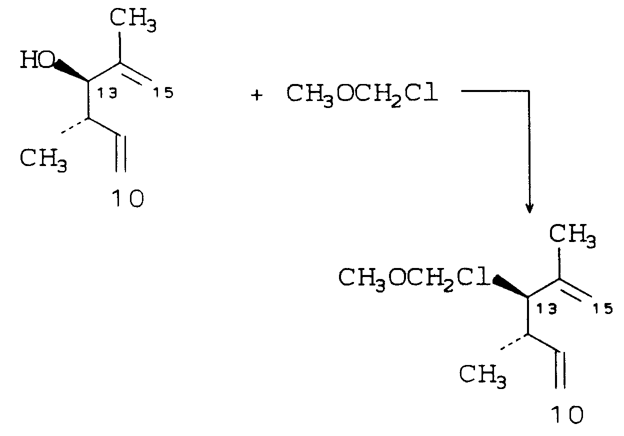
The chloromethyl methyl ether reagent is used in excess, about eleven equivalents, in order to increase the rate of reaction and to insure a good conversion. However, it is expected that a lower excess, from 1.1 to 11 equivalents, would also furnish the product. The preferred acid acceptor is N,N-diisopropylethylamine although other hindered non-nucleophilic trialkyl amines are acceptable. Triethylamine, although reactive with halo methyl ethers, is also satisfactory for this reaction. The amine is used in excess of the chloromethyl methyl ether, ranging from 10-18, preferably 13 equivalents of N,N-diisopropylethylamine. The preferred solvent for the chloromethyl methyl ethers is methylene chloride at a concentration of about 4 ml per gram of macrolide. Other aprotic solvents, such as chloroform, tetrahydrofuran and acetonitrile are satisfactory. Temperatures of from 10 to 120°C, preferably 10 to 60°C, and more preferably from 20 to 40°C are employed. The reaction is generally complete in from 12 to 24 hours.

Chloromethyl methyl ether is commerically available or it is readily prepared by the reaction of methanol with paraformaldehyde [(CH₂O)_{n'}) in the presence of hydrogen chloride, by a modification of the Henry synthesis as described by H. W. Lucien and C. T. Mason in J. Am. Chem. Soc., 71, 258 (1949). In addition, an improved process for the preparation of chloromethyl methyl ether from dimethoxy methane, methanol and acetyl chloride in situ is described in Amato et al., Synthesis, 970-971 (1979).

The 13-hydroxy epimers of the avermectin aglycones, the starting materials for the instant compounds, are prepared as outlined below and as described in US patent 4,587,247 to Linn et al. and in Mrozik et al., J. Med Chem. 32 pg 375 (1989). 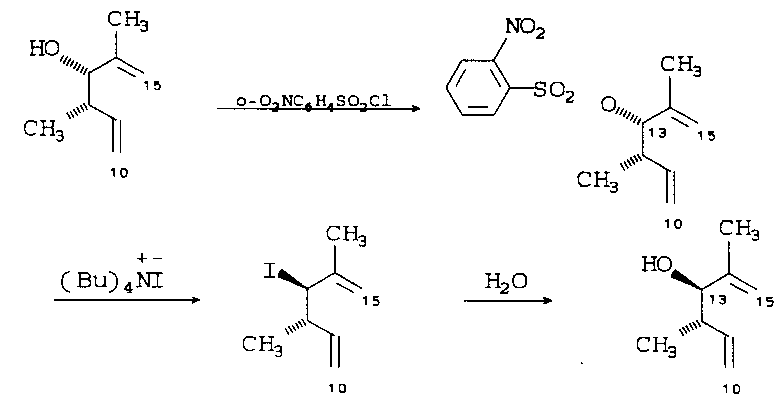
The normal protected avermectin aglycones wherein the 13-position substituent is hydroxy are treated with o-nitrobenzenesulfonyl chloride in the presence of a base such as N,N-diisopropylamine, triethylamine and the like and tetrabutylammoniumiodide furnishing the 13-β- (also called 13-epi-) iodo-13-deoxy avermectin aglycones. The iodo intermediates are heated at 100°C in 2,6-lutidine and water providing the 13β-avermectin aglycones. These 13β epimers are derivatized by the methods described above furnishing the same types of 13β-methoxymethyl avermectin aglycones. The 13-normal (the 13α) compound (top) and the 13-epi- (the 13β-) compound (bottom) are shown in the following partial structures.

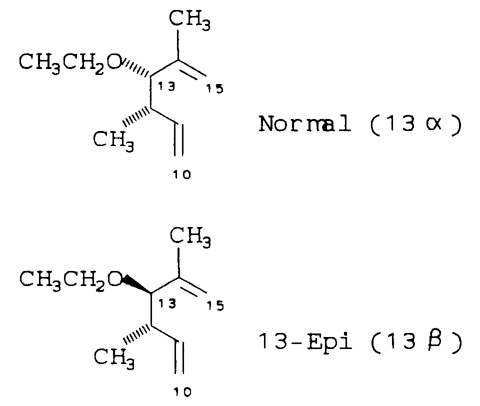

### DISCUSSION OF UTILITY

The novel compounds of this invention have significant parasiticidal activity as anthelmintics, ectoparasiticides, insecticides and acaricides, in human and animal health and in agriculture.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris and Parascaris. Certain of these, such as Nematodirus, Cooperia and Oesophagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections known as helminthiasis lead to anemia, malnutrition, weakness, weight loss, severe damage to the walls of the intestinal tract and other tissues and organs and, if left untreated, may result in death of the infected host. The substituted avermectin compounds of this invention have unexpectedly high activity against these parasites, and in addition are also active against Dirofilaria in dogs, Namatospiroides, Syphacia, Aspiculuris in rodents, arthropod ectoparasites of animals and birds such as ticks, mites, lice, fleas, blowfly, in sheep Lucilia sp., biting insects and such migrating diperous larvae as Hypoderma sp. cattle, Gastrophilus in horses, and Cuterebra sp. in rodents.

The instant compounds are also useful against parasites which infect humans. The most common genera of parasites of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunculus and extra intestinal stages of the intestinal worms Strongyloides and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp., and the housefly Musca domestica.

The compounds are also useful against insect pests of stored grains such as Tribolium sp., Tenebrio sp. and of agricultural plants such as2spider mites, (Tetranychus sp.), aphids, (Acyrthiosiphon sp.); against migratory orthopterans such as locusts and immature stages of insects living on plant tissue. The compounds are useful as a nematocide for the control of soil nematodes and plant parasites such as Meloidogyne spp. which may be of importance in agriculture The compounds are active against other plant pests such as the southern army worm and Mexican bean beetle larvae.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contains from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the avermectin derivatives in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or optionally fed separately. Alternatively, the antiparasitic compounds of our invention may be administered to animals parenterally, for example, by intraruminal, intramuscular, intratracheal, or subcutaneous injection in which event the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol formal, and aqueous parenteral formulations are also used. The active avermectin compound or compounds are dissolved or suspended in the parenteral formulation for administration; such formulations generally contain from 0.005 to 5% by weight of the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting insects in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by the oral administration of from about 0.001 to 10 mg per kg of animal body weight, such total dose being given at one time or in divided doses over a relatively short period of time such as 1-5 days. With the preferred compounds of the invention, excellent control of such parasites is obtained in animals by administering from about 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active hydrogenated avermectin compounds are intimately dispersed throughout the carrier by methods such as grinding, stirring, milling or tumbling. Compositions containing from about 0.005 to 2.0% by weight of the active compound are particularly suitable as feed premixes. Feed supplements, which are fed directly to the animal, contain from about 0.0002 to 0.3% by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular avermectin derivative employed, the compounds of this invention are usually fed at concentrations of between 0.00001 to 0.002% in the feed in order to achieve the desired antiparasitic result.

The avermectin compounds of this invention are also useful in combatting agricultural pests that inflict damage upon crops while they are growing or while in storage. The compounds are applied using known techniques as sprays, dusts, emulsions and the like, to the growing or stored crops to effect protection from such agricultural pests.

In using the compounds of this invention, the individual substituted avermectin components may be prepared and used in that form. Alternatively, mixtures of two or more of the individual avermectin components may be used, as well as mixtures of the parent avermectin compounds, other avermectin compounds or other active compounds not related to avermectin, with the compounds of this invention.

The following examples are provided in order that this invention might be more fully understood; they are not to be construed as limitative of the invention.

### EXAMPLES

The substituted avermectin derivatives prepared in the following examples are generally isolated as amorphous solids and not as crystalline solids. They are thus characterized analytically using techniques such as mass spectrometry, nuclear magnetic resonance, and the like. Being amorphous, the compounds are not characterized by sharp melting points, however, the chromatographic and analytical methods employed indicate that the compounds are pure.

Completion of reaction and purity of products were determined by thin layer chromatography (TLC) and high pressure liquid chromatography (HPLC). TLC was run using Analtech silica gel GF plates and developed with low percentages of methanol in methylene chloride. HPLC was run employing a Whatman Partisil PXS 10/25 ODS-3 reverse phase C₁₈ column and solutions containing low percentages of water in methanol with ultraviolet detection at 244 nm.

In the following examples, various starting materials are derivatives of avermectin compounds. The avermectin compounds and the preparation and isolation thereof from fermentation broths are described in United States Patent No. 4,310,519 issued 12 January 1982. The selectively hydrogenated 22,23-dihydro derivatives of avermectin compounds are described in U.S. Patent 4,199,569 issued 22 April 1980. The aglycone derivatives of avermectin compounds are described in U.S. Patent 4,206,205.

### EXAMPLE 1

### 5-O-t-Butyldimethylsilyl-22,23-dihydro avermectin B1a/B1b aglycones

t-Butyldimethylsilyl chloride, 4.41 g, was added rapidly to a stirred solution containing 7.64 g of 22,23-dihydro avermectin B1a/B1b aglycones and 4.15 g of imidazole in 23 ml of dry dimethylformamide at room temperature, 22°C. After 3 hours the reaction mixture was poured into 200 ml of water followed by extraction with ether. The ether solution was extracted with water, dried over anhydrous sodium sulfate and evaporated leaving 10.3 g of solid residue. This product was purified by column chromatography using silica gel and methylene chloride furnishing 8.6 g of 5-O-t-butyldimethylsilyl-22,23-dihydro avermectin B1a/B1b aglycones as an amorphous solid. The structure was confirmed by nuclear magnetic resonance, mass spectrometry, ultra-violet and elemental analyses.

### EXAMPLE 2

### 13β-Iodo-13-deoxy-5-O-t-butyldimethylsilyl-22,23-dihydro avermectin B1a/B1b aglycones

A solution containing 1.05 g (4.7 mmol) of o-nitrobenzenesulfonyl chloride in 15 ml of methylene chloride was added dropwise over about 30 minutes with stirring to a solution containing 1.00 g (1.43 mmol) of 5-O-t-butyldimethylsilyl-22,23-dihydro avermectin B1a/B1b aglycones, 1.1 ml (6.3 mmol) of N,N-diisopropylethylamine, 770 mg (6.3 mmol) of 4-dimethylaminopyridine and 2.0 g (5.4 mmol) of tetrabutylammonium iodide in 20 ml of methylene chloride at room temperature (23°C). Stirring was continued for 3.5 hours. The reaction mixture was poured into ice water and diluted with ether. The ether layer was separated and the aqueous layer was extracted with ether. The ether solutions were combined, extracted with water and sodium chloride solutions and dried over magnesium sulfate. Evaporation of the solvent left 1.5 g of residue which was chromatographed on silica gel using methylene chloride. 13β-Iodo-13-deoxy-5-O-t-butyldimethylsilyl-22,23-dihydro avermectin B1a/B1b aglycones, 540 mg of yellow foam solid, was obtained. The structure was confirmed by nuclear magnetic resonance and mass spectrometry.

### EXAMPLE 3

### 13β-5-O-t-Butyldimethylsilyl-22,34-dihydro avermectin B1a/B1b aglycones

A solution containing 902 mg of 13β-iodo-13-deoxy-5-O-t-butyldimethylsilyl-22,23-dihydro avermectin B1a/B1b aglycones from Example 2 in 5.3 ml 2,6-lutidine and 135 µl of water was sealed under nitrogen and heated at 100°C for 14.5 hours until no more starting material remained as determined by TLC. The solution was evaporated in vacuo. The residue was dissolved in toluene and evaporated in vacuo. This step was repeated several times in order to remove residual lutidine. The solid residue was extracted with ethyl ether. The insolubles were discarded and the filtrate evaporated in vacuo leaving 1.1 g of solids. These solids were chromatographed on a column of silica gel using methylene chloride-methanol (98:2). Two major bands were separated. The slower moving band, 370 mg, was chromatographed on a silica gel column using methylene chloride-methanol (99:1) furnishing 245 mg of 13β-5-O-t-butyldimethylsilyl-22,23-dihydro avermectin B1a/B1b aglycones as an amorphous solid, purity 97% by HPLC. The structure was determined by nuclear magnetic resonance and mass spectrometry.

### EXAMPLE 4

### 13β-O-Methoxymethyl-5-O-t-butyldimethylsilyl-22,23-dihydro avermectin B1a/B1b aglycones

2.0 g of 5-O-tert-butyldimethylsilyl-13β-22,23-dihydro avermectin B1a/B1b aglycones (0.00286 mol, 1 eq.) was dissolved in. 20 mL of dry methylene chloride under nitrogen at room temperature and stirred magnetically. 6.00 mL of N,N-diisopropylethylamine (4.45 g, 0.0345 mol, 12 eq.) was added followed by 4.8 mL of chloromethyl methyl ether solution containing 2.30 g (0.0 286 mol), prepared according to Amato et al. Synthesis 1979, 970 (See below). The solution was added dropwise, and a slight heat rise was noted. The reaction was followed by TLC (SiO₂, 25% ethylacetate-hexane). The reaction was stirred at room temperature overnight under nitrogen, whereupon TLC showed complete reaction. A solution of 0.5 mole - 7.9 g of Na₂S₂O₃ in 30 ml of water was added to the reaction mixture and it was stirred at room temperature for 30 minutes in order to destroy unreacted chloromethyl methyl ether. The solution was transferred into a separatory funnel and the layers were separated, the organic phase was washed twice with 40 ml of saturated sodium bicarbonate solution, then water, dried over sodium sulfate and concentrated in vacuo under high vacuum to a brown oil. The brown oil was purified on a column of 100 g of silica gel with 5 to 10% ethyl acetate:hexane to give 1.9 g of pure 13β-O-methoxymethyl-5-O-t-butyldimethylsilyl-22,23-dihydro avermectin B1a/B1b aglycones . The structure was confirmed by nuclear magnetic resonance and mass spectrometry.

### Preparation of chloromethyl methyl ether free of Bis[chloromethyll ether.

Acetyl chloride, 35.3 mL (0.49 mol), was added in three portions to a solution of sieve dried dimethoxymethane, 45 mL (0.51 mol) and anhydrous methanol, 1.2 mL (0.49 mol) with stirring under nitrogen at room temperature. The temperature of the reaction solution rose from 16° to 25°C during the addition. After standing for 36 hr at room temperature, the solution contained 6 mmol of chloromethyl methyl ether per mL. Approximately 83 mL. (0.5 mol) were prepared. For details see J.S. Amato et. al., Synthesis, 1979, 970-971.

An efficient hood and special precautions to avoid contacting the reagent or breathing its vapors are required for the preparation and handling of this reagent.

### EXAMPLE 5

### 13β-O-methoxymethyl-22,34-dihydro avermectin B1a/B1b aglycones

81 Mg of 13β-O-methoxymethyl-5-O-t-butyldimethylsilyl-22,23-dihydro avermectin B1a/B1b aglycones from Example 4 in 5.0 ml of 0.5% para-toluenesulfonic acid monohydrate in methanol was stirred at room temperature (23°C) until the reaction was complete as determined by TLC. After 50 minutes, the reaction solution was diluted with methylene chloride and extracted with dilute aqueous sodium bicarbonate. The methylene chloride solution was dried over anhydrous sodium sulfate and evaporated under reduced pressure. The residue was chromatographed on two 500µ 20X20 cm silica gel GF plates using methylene chloride:methanol (95:5) furnishing 13β-O-methoxymethyl-22,23-dihydro avermectin B1a/B1b aglycones as an amorphous solid, E(1 cm, 1%) at λmax (MeOH) 450nm. The structure was confirmed by nuclear magnetic resonance, mass spectrometry, ultra-violet and elemental analyses.

## Claims

1. A compound having the formula: 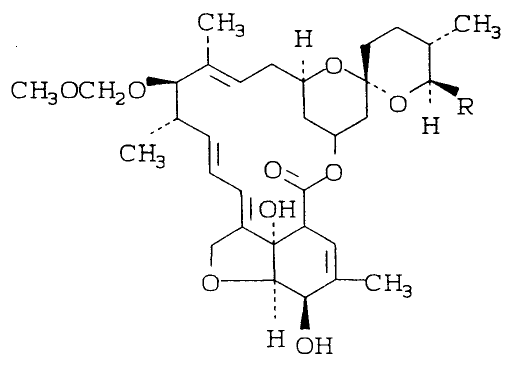 where R is sec-butyl or isopropyl.

2. A process for the preparation of the compounds of Claim 1 which comprises treating the corresponding compound wherein the substituent in the 13-position is β-hydroxy with CH₃OCH₂Cl in the presence of base.

3. A pharmaceutical composition for treatment of animals infected with parasites which comprises a therapeutically effective dosage amount of a compound of Claim 1 and a pharmaceutically acceptable carrier.

4. The use of a compound of Claim 1 for the manufacture of a medicament for treating an animal suffering from a parasitic condition.
